(19) Europäisches Patentamt
European Patent Office
Office européen des brevets

(11) Veröffentlichungsnummer: **0 035 714**
**B1**

# (12) EUROPÄISCHE PATENTSCHRIFT

(45) Veröffentlichungstag der Patentschrift: **31.10.84**

(51) Int. Cl.³: **C 07 D 295/14**, C 07 D 243/08, A 61 K 31/395 // C07C121/66

(21) Anmeldenummer: **81101422.4**

(22) Anmeldetag: **27.02.81**

(54) **10-substituierte 5-Cyanmethylen-10,11-dihydro-dibenzo-(a,d)-cycloheptene, Verfahren zu ihrer Herstellung und diese enthaltende therapeutische Mittel.**

(30) Priorität: **08.03.80 DE 3009045**

(43) Veröffentlichungstag der Anmeldung: **16.09.81 Patentblatt 81/37**

(45) Bekanntmachung des Hinweises auf die Patenterteilung: **31.10.84 Patentblatt 84/44**

(84) Benannte Vertragsstaaten: **AT BE CH DE FR GB IT LI NL SE**

(56) Entgegenhaltungen:
**CA - A - 1 000 701**
**DE - A - 1 568 089**
**DE - A - 1 620 151**
**DE - A - 2 918 778**

**ARZNEIMITTELFORSCHUNG, Band 25, Nr. 5, Veröffentlicht in 1975, Seiten 712-720 Aulenburg, DE. J. SCHMUTZ: "Neuroleptic piperazinyl-dibenzo-azepines"**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Steiner, Gerd, Dr., Oberer Waldweg 1, D-6719 Kirchheim (DE)**
Erfinder: **Hofmann, Hans Peter, Dr., Untere Hart 12, D-6703 Limburgerhof (DE)**
Erfinder: **Kreiskott, Horst, Dr., Am Boehlig, D-6706 Wachenheim (DE)**
Erfinder: **Teschendorf, Hans-Juergen, Dr., Rene-Bohn-Strasse 4, D-6700 Ludwigshafen (DE)**

EP 0 035 714 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft in 10-Stellung substituierte 5-Cyanmethylen-10,11-dihydro-dibenzo[a,d]-cycloheptene, und ihre pharmazeutisch verträglichen Säureadditionssalze, Verfahren zu ihrer Herstellung und diese enthaltende Arzneimittel, insbesondere als Sedativa, Hypnotika oder Tranquilizer.

Es ist bekannt, daß tricyclische Ringsysteme mit einer Dibenzo-struktur zu einem zentralen heterocyclischen 7-Ring, der gegebenenfalls einen basischen Seitenrest, wie beispielsweise einen N-Methylpiperazinrest aufweist, neuroleptische Wirkungen aufweisen können. Solche Tricyclen sind beispielsweise N-Methylpiperazin-Derivate von

Dibenzo[b,e][1,4]-diazepinen (Clozapine),
Dibenzo[b,f][1,4]-thiazepinen (Clotiapine),
Dibenzo[b,f][1,4]-oxazepinen (Loxapine) oder Morphantridinen (Perlapine), wie beispielsweise aus der Zusammenfassung von J. Schmutz in der Arzneimittelforschung 25, 712—720 (1975) hervorgeht.

In der EP-A-19 172 werden 6-substituierte 11-Alkylenmorphantridine mit wertvollen pharmakologischen Eigenschaften vorgeschlagen. Gegenstand dieser Anmeldung sind Derivate mit einem abgewandelten Ringsystem, die ein unterschiedliches pharmakologisches Wirkprofil aufweisen.

Aus DE-A-1 568 089 ist zu entnehmen, daß in 5-Stellung durch Alkylamin substituierte 10,11-Dihydro-dibenzo[a,d]-cycloheptene (Beispiel 2) psychotrope Eigenschaften aufweisen. Weiterhin kennt man auch 10-Piperazinodibenzo[1,d]-cycloheptadienderivate (Anspruch 1) mit sedativer, antidepressiver und spasmolytischer Wirksamkeit und aus DE-A-1 620 151 ebenso Dibenzocycloheptadienderivate mit gleicher Wirkungsrichtung. Die bekannten Substanzen genügen jedoch nicht allen Anforderungen.

Es wurde nun gefunden, daß 10-substituierte 5-Cyanmethylen-10,11-dihydro-dibenzo[a,d]-cycloheptene der allgemeinen Formel (I)

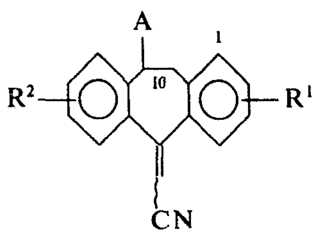

(I)

in der $R^1$ und $R^2$ Wasserstoffatome, Halogenatome, Alkylreste mit 1 bis 3 C-Atomen oder Trifluormethyl bedeuten, und für A ein Piperazinyl- oder Homopiperazinylrest steht, wobei das 2. Stickstoffatom gegebenenfalls durch einen Alkylrest mit 1 bis 3 C-Atomen, 2-Hydroxyäthyl, einen Cycloalkyl- oder Cycloalkylmethylrest mit 3 bis 7 C-Atomen im Cycloalkylring, einen Prop-2-inylrest und gegebenenfalls zusätzlich durch Sauerstoff in Form eines N-Oxids substituiert ist, und ihre physiologisch verträglichen Säureadditionssalze.

Gegenstand der EP-A 35 711 mit gleicher Priorität sind die entsprechenden, in 10,11-Stellung nicht hydrierten Cycloheptenderivate.

Für die Reste $R^1$ und $R^2$ seien hervorgehoben Wasserstoff, Fluor, Chlor, Methyl und Trifluormethyl, wovon die Bedeutungen Wasserstoff und Chlor besonders bevorzugt sind.

Besonders bevorzugte Reste A sind der 4-Methyl-piperazinyl-, 4-Methyl-4-oxy-piperazinyl-, 4-Cyclopropyl-piperazinyl-, 4-Cyclopropylmethyl-piperazinyl-, 4-Propin-2-yl-piperazinyl-, 4-(2-Hydroxy)-äthyl-piperazinyl-, 4-Äthyl-piperazinyl- und der N-Methyl-homopiperazinyl-Rest.

Es wird darauf hingewiesen, daß die erfindungsgemäßen Verbindungen der Formel I als cis-trans-Isomere Ia und b vorliegen.

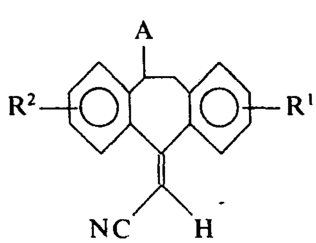

(Ia)

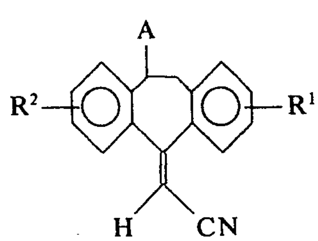

(Ib)

Gegebenenfalls können die cis-trans-Isomeren, in der Regel handelt es sich um Gemische im Verhältnis 1 : 1, beispielsweise durch fraktionierte Kristallisation oder durch Säulenchromatographie getrennt werden.

Aufgrund der oben genannten Bedeutungen sind die folgenden Verbindungen als besonders bevorzugt und wirksam zu nennen:

cis,trans-5-Cyanmethylen-10-(4-methyl-piperazin-1-yl)-10,11-dihydro-dibenzo[a,d]-cyclohepten,
cis,trans-5-Cyanmethylen-10-(4-methyl-4-oxy-piperazin-1-yl)-10,11-dihydro-dibenzo[a,d]-cyclohepten,
cis,trans-5-Cyanmethylen-2-chlor-10-(4-methyl-piperazin-1-yl)-10,11-dihydro-dibenzo[a,d]-cyclohepten,
cis,trans-5-Cyanmethylen-10-(4-β-hydroxyäthyl-piperazin-1-yl)-10,11-dihydro-dibenzo[a,d]-cyclohepten,
cis,trans-5-Cyanmethylen-10-(4-äthyl-piperazin-1-yl)-10,11-dihydro-dibenzo[a,d]-cyclohepten,
cis,trans-5-Cyanmethylen-10-(N'-methyl-homopiperazin-1-yl)-10,11-dihydro-dibenzo[a,d]-cyclohepten.

Die erfindungsgemäßen Verbindungen der Formel I werden hergestellt, indem man eine Verbindung der Formel (II)

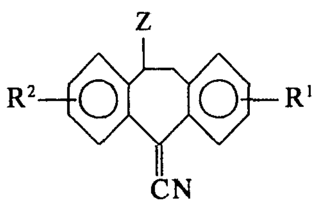

(II)

in der $R^1$ und $R^2$ die für Formel I angegebenen und bevorzugten Bedeutungen haben und Z Brom oder Chlor darstellt, mit einem Nukleophil AH, in dem A die für Formel (I) angegebenen Bedeutungen hat, umsetzt und gegebenenfalls die erhaltene Verbindung in das N-Oxid und/oder in das Säureadditionssalz einer physiologisch verträglichen Säure überführt.

Die Umsetzung erfolgt zweckmäßig in Gegenwart einer überschüssigen Menge des verwendeten Amins AH in einem dipolaren aprotischen Lösungsmittel, vorzugsweise Dimethylformamid, in Gegenwart von ungefähr ⅓ Moläquivalent eines einwertigen Salzes von Silber oder Kupfer, vorzugsweise von Silbernitrat, bei Temperaturen zwischen Raumtemperatur und 80°C und ist im allgemeinen innerhalb von 0,5 bis 4 Stunden beendet. Gegebenenfalls ist der Ausschluß des Sauerstoffs der Luft und das Arbeiten unter Inertgas, beispielsweise unter Stickstoff, von Vorteil.

Bei den Umsetzungen wird das Nukleophil AH vorteilhafterweise in mindestens 2fachem molarem bis 20molarem Überschuß verwendet.

Zweckmäßig wird das bei den Umsetzungen als Nebenprodukt durch Halogenwasserstoff-Eliminierung entstehende 5-Cyanmethylen-dibenzo[a,d]-cyclohepten durch Säulenchromatographie abgetrennt.

Die Überführung einer Verbindung der Formel (I) in das N-Oxid erfolgt in üblicher Weise, zweckmäßig mit wäßrigem Wasserstoffperoxid (30 Gew.-%) in äthanolischer Lösung. Die Überführung in das Säureadditionssalz einer physiologisch verträglichen Säure erfolgt ebenso in üblicher Weise.

Die Ausgangsverbindungen der Formel (II) werden hergestellt, indem man ein 5-Cyanmethylen-10,11-dihydro-dibenzo[a,d]-cyclohepten der Formel (III)

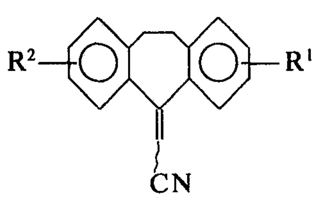

(III)

in der $R^1$ und $R^2$ die für Formel I angegebenen Bedeutungen haben, mit 1 Mol N-Brom-(Chlor)-succinimid in einem Halogenkohlenwasserstoff bei Temperaturen von 50 bis 100°C zum 10-Brom-(Chlor)-5-cyanmethylen-10,11-dihydro-dibenzo[a,d]-cyclohepten der Formel II umsetzt.

Die 5-Cyanmethylen-10,11-dihydro-dibenzo[a,d]-cycloheptene der Formel III werden durch Carbonyl-Olefinierung hergestellt, indem man ein literaturbekanntes Dibenzosuberon der Formel (IV) (vgl. E. L. Engelhardt et al., J. Med. Chem. 8, 829 [1965])

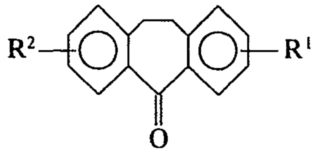

(IV)

in der $R^1$ und $R^2$ die für Formel (I) angegebenen Bedeutungen haben, mit einem Phosphonat der Formel (IVa)

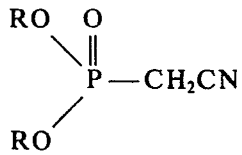

(IVa)

in der R einen Alkylrest mit 1 bis 3 C-Atomen bedeutet, unter den Bedingungen der Wittig-Horner-Reaktion in einem inerten Lösungsmittel, besonders bevorzugt Dimethylformamid, in Gegenwart von einem Moläquivalent einer Base, bevorzugt Natriumalkoholat, Natriumhydrid oder Natriumamid, bei Temperaturen von 20 bis 80°C umsetzt oder mit einem Phosphoniumsalz der Formel (IVb)

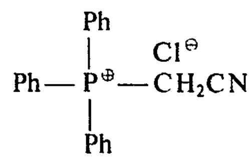

(IVb)

in der Ph für einen Phenylrest steht, unter den Bedingungen der klassischen Wittig-Reaktion in einem aprotischen organischen Lösungsmittel, insbesondere einem gesättigten aliphatischen oder gesättigten cyclischen Äther, wie Diäthyläther, Tetrahydrofuran oder Dioxan, oder vorzugsweise in Dimethylformamid, in Gegenwart von einem Moläquivalent einer Base, insbesondere eines Alkalialkoholats, vorzugsweise Natriummethylat oder Natriumäthylat, oder Natriumhydrid, Natriumamid oder einer metallorganischen Verbindung, wie Butyllithium, bei Temperaturen von 20 bis 100°C umsetzt.

Neben den in den Beispielen aufgeführten Verbindungen seien weiterhin folgende Verbindungen beispielhaft genannt:

cis,trans-8-Chlor-5-cyanmethylen-10-(4-methyl-piperazin-1-yl)-10,11-dihydro-dibenzo[a,d]-cyclohepten,
cis,trans-5-Cyanmethylen-10-(4-cyclopropyl-piperazin-1-yl)-10,11-dihydro-dibenzo[a,d]-cyclohepten,
cis,trans-5-Cyanmethylen-10-(4-cyclopropylmethyl-piperazin-1-yl)-10,11-dihydro-dibenzo[a,d]-cyclohepten,
cis,trans-5-Cyanmethylen-10-(4-propin-2-yl-piperazin-1-yl)-10,11-dihydro-dibenzo[a,d]-cyclohepten.

Die erfindungsgemäßen Verbindungen der Formel (I) werden in der Regel in Form von gelblichen bis gelben Kristallen erhalten und können durch Umkristallisation aus den üblichen organischen Lösungsmitteln, bevorzugt aus einem niederen Alkohol, wie Äthanol, umkristallisiert oder durch Säulenchromatographie gereinigt werden.

Die freien 10-substituierten 5-Cyanmethylen-10,11-dihydro-dibenzo[a,d]-cycloheptene der Formel (I) können in üblicher Weise in das Säureadditionssalz einer pharmakologisch verträglichen Säure überführt werden, vorzugsweise durch Versetzen einer Lösung mit einem Äquivalent der entsprechenden Säure. Als übliche physiologisch verträgliche anorganische Säuren kommen beispielsweise in Betracht Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure oder können aus Fortschritte der Arzneimittelforschung Band 10, Seiten 224 bis 225, Birkhäuser Verlag, Basel und Stuttgart, 1966, entnommen werden.

Die erfindungsgemäßen Verbindungen weisen wertvolle pharmakologische Eigenschaften auf. Sie können als Sedativa, Tranquilizer, Hypnotika, Neuroleptika oder Antidepressiva Verwendung finden. Mehrere der genannten Wirkungsqualitäten können kombiniert bei einer erfindungsgemäßen Verbindung auftreten.

Nach den Ergebnissen der durchgeführten pharmakologischen Experimente eignen sich die erfindungsgemäßen Substanzen in Anbetracht ihrer sedativen-tranquillisierenden, muskelrelaxierenden und antimonaminergen Wirkung insbesondere als Sedative, Hypnotika, Minor- oder Major-Tranquilizer,

und Neuroleptika.

Zur Wirkungsanalyse fanden folgende Methoden Verwendung:

### 1. Sedative Wirkung

4 bis 8 Gruppen von jeweils 3 weiblichen NMRI-Mäusen erhalten die Substanzen oral appliziert. Die photoelektrische Bestimmung der durch eine neue Umgebung induzierten Orientierungshypermotilität erfolgt 30 Minuten nach der Applikation der Substanzen für eine Dauer von 30 Minuten.

Als $ED_{50}$ wird die Dosis bestimmt, welche im Vergleich mit Placebo behandelten Kontrolltieren eine Abnahme der Orientierungshypermotilität um 50% bewirkt.

### 2. Muskelrelaxierende Wirkung

Die Messung beruht auf der Quantifizierung des tonischen Dehnungsreflexes am Gastrocnemius des Kaninchen (Teschendorf et al., Arch. Pharmacol. exp. Path. 266, 462, 1970). Das Kaninchen wird in einer speziellen Apparatur fixiert, die es erlaubt, die Pfote im Sprunggelenk in definierter und reproduzierbarer Weise zu beugen. Durch die Beugung wird in der Wadenmuskulatur ein tonischer Dehnungsreflex ausgelöst. Die elektrische Aktivität des Muskels während der Kontraktion wird registriert und die Einzelimpulse gezählt. Die Dehnung (Dauer 5 s) wird in Minutenabständen wiederholt. Nach Erreichen einer konstanten Impulszahl (Kontrollwert) wird die Testsubstanz intravenös appliziert. Die Impulszahlen nach Applikation werden auf die Vorwerte bezogen. Tierzahl pro untersuchter Dosis 4 bis 6. Die $ED_{50}$ ist die Dosis, die die Muskelaktivität — bezogen auf den Vorwert — um die Hälfte reduziert.

### 3. Antimethamphetamin-Wirkung

Methamphetamin (2,5 mg/kg i.v.) ruft an Ratten regelmäßig folgende Symptome hervor: motorische Unruhe, Such- und Schnüffelbewegungen, gesträubtes Fell sowie Tremor (Janssen et al., Arzneim.-Forsch./Drug Res. 13, 205, 1963; Randrug et al., Psychopharmacologia 11, 300, 1967). Die Prüfsubstanzen werden 30 min vor Methamphetamin intraperitoneal verabfolgt. Kriterium für einen Substanzeffekt ist das Ausbleiben der Schnüffelbewegungen innerhalb eines 5minütigen Beobachtungszeitraums nach Methamphetamininjektion. Als mittlere Hemmdosis ($ED_{50}$) wird mittels Probitanalyse die Dosis ermittelt, die das Symptom bei der Hälfte der Tiere verhindert. Tierzahl pro untersuchter Dosis: 10.

### 4. Antiapomorphin-Wirkung

An Gruppen von 4 bis 6 weiblichen Sprague-Dawley-Ratten werden durch subcutane Applikation von 1,5 mg/kg Apomorphin Kieferbewegungen ausgelöst, die über implantierte Elektroden registriert werden (Mandibulogramm nach Kubacki, Psychopharmacology 59, 209, 1978). Die Prüfsubstanzen werden 90 Minuten vor Apomorphin oral appliziert. Als $ED_{50}$ wird die Dosis bestimmt, welche die Anzahl der Kieferbewegungen im Vergleich zu placebobehandelten Kontrolltieren um 50% reduziert.

### 5. Anticholinerge Wirkung

Gruppen von 10 weiblichen NMRI-Mäusen erhalten Physostigmin in einer letalen Dosis (0,825 mg/kg) subcutan. Die Prüfsubstanzen werden 30 Minuten vor der Physostigminapplikation oral appliziert.

Als $ED_{50}$ wird die Substanzdosis bestimmt, welche 50% der Tiere vor dem Tod durch Physostigmin schützt.

### 6. Akute Toxizität

Gruppen von 5 bis 10 weiblichen NMRI-Mäusen erhalten die Substanzen i.p. Als $LD_{50}$ wird die Dosis ermittelt, nach welcher 50% der behandelten Tiere sterben.

In diesen Experimenten (vgl. Tab. 1) wurden auffallend sedativ-hypnotische Wirkungen bei den erfindungsgemäßen Substanzen nachgewiesen, die etwa gleich stark ausgeprägt sind wie bei den Referenzsubstanzen Clozapin und Perlapin. Ebenso findet sich eine muskelrelaxierende Wirkung, welche diejenige der Vergleichssubstanzen zum Teil deutlich übertrifft (Verbindungen Beispiel 4).

Als Parameter für eine neuroleptische Qualität kann eine antimonaminerge Wirkung — hier gemessen am Methamphetamin- bzw. Apomorphinantagonismus — gewertet werden. Die Vergleichsverbindungen Clozapin und Perlapin werden in der Wirkstärke erreicht oder deutlich – bis zu 7fach – übertroffen. Im Gegensatz zu Clozapin besitzen die neuen Verbindungen – wie der Antiphysostigmintest an der Maus zeigt – keine anticholinergen Eigenschaften, woraus auf geringere periphere Nebenwirkungen bei therapeutischer Anwendung zu schließen ist.

Auf Grund der pharmakologischen Befunde können die erfindungsgemäßen Verbindungen in entsprechenden galenischen Zubereitungen als Sedativa Hypnotika, Minor- oder Major-Tranquillizer und Neuroleptika verwendet werden.

Tabelle 1

| Beispiel Nr. | Sedation | | Muskel-relaxation | | Anti-methamphetamin-Wirkung | | Antiapo-morphin-Wirkung | | Anticho-linerge Wirkung | Toxi-zität |
|---|---|---|---|---|---|---|---|---|---|---|
| | $ED_{50}$ | R.W.[1] | $ED_{50}$ | R.W. | $ED_{50}$ | R.W. | $ED_{50}$ | R.W. | $LD_{50}$ | $LD_{50}$ |
| 1 | 5,6 | 0,85 | 0,1 | 0,46 | 5,4 | 6,85 | | | >46,4 | >100 |
| 7 | 10,0 | 0,47 | | | 30 | 1,23 | | | >100 | >100 |
| 5 | 6,7 | 0,71 | 0,1 | 0,46 | 13 | 2,85 | | | >46,4 | >100 |
| 8 | 15,2 | 0,31 | | | 39 | 0,95 | 7 | 1,14 | >100 | >100 |
| 4 | 5,5 | 0,86 | 0,02 | 2,30 | 4,6 | 8,04 | | | | |
| Clozapin | 4,74 | 1,0 | 0,046 | 1,00 | 37 | 1,00 | 8 | 1,00 | 14,1 | 215 |
| Perlapin | 2,01 | 2,36 | 0,1 | 0,46 | 31 | 1,19 | 21,5 | 0,37 | >21,5 | 215 |

[1]) R.W. = relative Wirksamkeit.

Die Erfindung betrifft dementsprechend auch ein therapeutisches Mittel, gekennzeichnet durch einen Gehalt an einer Verbindung der Formel (I) oder deren pharmakologisch verträgliches Säureadditionssalz als Wirkstoff neben üblichen Träger- und Verdünnungsmitteln.

Therapeutische Mittel mit üblichen Trägerstoffen oder Verdünnungsmitteln und den üblicherweise verwendeten technischen Hilfsstoffen können entsprechend der gewünschten Applikationsart und mit einer zur Anwendung geeigneten Dosiereinheit in an sich üblicher Weise hergestellt werden. Als Einzeldosen beim Menschen kommen 10 bis 100 mg in Betracht.

Die neuen Verbindungen können in den üblichen galenischen Applikationsformen, fest oder flüssig, angewendet werden, wie Tabletten, Kapseln, Pulver, Granulate, Dragees oder Lösungen. Diese werden in üblicher Weise hergestellt. Die Wirkstoffe können dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wäßrigen oder nicht wäßrigen Trägern, Netzmitteln, Dispergiermitteln, Emulgatoren und/oder Konservierungsmitteln verarbeitet werden (vgl. L. G. Goodman, A. Gilman, The Pharmacological Basis of Therapeutics). Die so erhaltenen Präparate enthalten den Wirkstoff normalerweise in einer Menge von 0,001 und 99 Gew.-%.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Puver, Lösungen, Suspensionen oder Depotformen. Es kommen auch parenterale Zubereitungen, wie Inkektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmittel, wie Maisstärke oder Alginsäure, Bindemittel wie Stärke oder Gelatine, Gleitmittel wie Magnesiumstearat oder Talk und/oder Mittel zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle

aus mehreren Schichten bestehen, wobei die oben bei den Tabletten erwähnten Hilfsstoffe verwendet werden können.

Lösungen oder Suspensionen mit den erfindungsgemäßen Wirkstoffen können zusätzlich geschmacksverbessernde Mittel, wie Vanillin oder Orangenextrakt, enthalten. Sie können außerdem Suspendierstoffe, wie Natriumcarboxymethylcellulose, oder Konservierungsstoffe, wie p-Hydroxybenzoate, enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Geeignete Suppositorien lassen sich beispielsweise durch Vermischen mit dafür vorgesehenen Trägermitteln, wie Neutralfetten oder Polyäthylenglykol bzw. deren Derivaten herstellen.

Die folgenden Beispiele dienen zur Erläuterung der Erfindung.

## Beispiel 1

cis,trans-5-Cyanmethylen-10-(4-methyl-piperazin-1-yl)-10,11-dihydro-dibenzo[a,d]-cyclohepten · ½ $H_2O$

1,0 g (5,9 mM) Silbernitrat werden in 10 ml abs. Dimethylformamid unter Rühren gelöst und mit einem großen Überschuß (5 bis 50 ml) N-Methylpiperazin versetzt. Anschließend läßt man sofort eine Lösung aus 7,0 g (22,6 mM) 10-Brom-5-cyanmethylen-10,11-dihydro-dibenzo[a,d]-cyclohepten (cis,trans-Isomerengemisch) in 35 ml Dimethylformamid zutropfen (leichte Erwärmung des Reaktionsgemisches auf 28 bis 30°C zeigt die exotherme Reaktion an, die Farbe wechselt von gelb nach grau durch ausgefälltes Silber) und läßt das Reaktionsgemisch 1 bis 3 Stunden bei Raumtemperatur unter Stickstoff rühren. Anschließend gießt man das Reaktionsgemisch auf 2 l Eiswasser (gutes Rühren erforderlich) und saugt die ausgefallenen grauen Festkörper nach einstündigem Nachrühren ab. Das Rohprodukt, das durch feinverteiltes Silber grau gefärbt ist, wird in wenig Methylenchlorid dispergiert und direkt durch Säulenchromatographie (Kieselgel, Methylenchlorid/Methanol 95/5, Entfernung des als Nebenprodukt entstandenen 5-Cyanmethylen-dibenzo[a,d]-cycloheptens) gereinigt. Man isoliert 1,5 g (20%) Produkt als cis,trans-Isomerengemisch, das anschließend noch aus Äthanol umkristallisiert werden kann. Farblose Kristalle mit Schmp. 141—143°C.

## Beispiel 2 (Ausgangsmaterial)

cis,trans-10-Brom-5-cyanmethylen-10,11-dihydro-dibenzo[a,d]-cyclohepten

Zur Herstellung des Vorproduktes 10-Brom-5-cyanmethylen-10,11-dihydro-dibenzo[a,d]-cyclohepten löst man 13,0 g (56 mM) 5-Cyanmethylen-10,11-dihydro-dibenzo[a,d]-cyclohepten in 200 ml Tetrachlorkohlenstoff und gibt 10,0 g (56 mM) N-Bromsuccinimid sowie 100 mg Benzoylperoxid hinzu. Man läßt das Reaktionsgemisch 3 Stunden am Rückfluß kochen und saugt nach dem Abkühlen das gebildete Succinimid ab. Nach Einengen der Mutterlauge erhält man das Rohprodukt als Kristall-Öl-Gemisch, das aus Äthanol umkristallisiert wird. Man isoliert 16,0 g (92%) Produkt mit Schmp. 148—150°C.

## Beispiel 3 (Ausgangsmaterial)

cis,trans-5-Cyanmethylen-10,11-dihydro-dibenzo[a,d]-cyclohepten

Zur Herstellung des Vorproduktes 5-Cyanmethylen-10,11-dihydro-dibenzo[a,d]-cyclohepten führt man eine Carbonyl-Olefinierung mit Hilfe der Wittig-Horner-Reaktion oder über die klassische Wittig-Synthese durch:

20,0 g (96 mM) Dibenzosuberon werden in 200 ml Dimethylformamid unter Erwärmung gelöst und unter Stickstoff gerührt. Man läßt dann gleichzeitig 20,3 g (115 mM) Diäthyl-cyanmethyl-phosphonat und 20,1 g (115 mM) Natriummethylat (30%) gelöst in 100 ml Dimethylformamid langsam zutropfen (Farbzunahme und Temperaturerhöhung zeigen den Beginn der Wittig-Reaktion an). Nach 12stündigem Nachrühren bei Raumtemperatur gießt man das Reaktionsprodukt auf Eiswasser, läßt noch 3 Stunden zur vollständigen Kristallisation gut rühren und saugt die ausfallenden Festkörper ab. Nach gutem Nachwaschen mit Wasser wird das Rohprodukt getrocknet und aus Äthanol umkristallisiert. Ausbeute: 14,0 g (63%) farblose Kristalle mit Schmp. 105—107°C.

Klassisches Wittig-Verfahren: Man legt Triphenylcyanomethyl-phosphonium-chlorid in Dimethylformamid vor, läßt anschließend 1 Moläquivalent einer 30%igen Natriummethylat-Lösung zutropfen oder versetzt mit 1 Moläquivalent Natriumhydrid und fügt zuletzt 1 Moläquivalent einer Lösung von Dibenzosuberon in Dimethylformamid hinzu. Man läßt das Reaktionsgemisch 5 bis 8 Stunden bei 50 bis 80°C nachrühren, gießt anschließend auf Eiswasser und extrahiert mehrmals mit Methylenchlorid.

Nach dem Trocknen und Einengen der organischen Phase kristallisiert man das Rohprodukt aus Äthanol um. Ausbeute: 45%, farblose Kristalle mit Schmp. 104—107°C.

Beispiel 4

cis,trans-2-Chlor-5-cyanmethylen-10-(4-methyl-piperazin-1-yl)-10,11-dihydro-dibenzo[a,d]-cyclohepten

a) cis,trans-2-Chlor-5-cyanmethylen-10,11-dihydro-dibenzo[a,d]-cyclohepten: Synthese analog Beispiel 3 mit unterschiedlicher Aufarbeitung durch Extraktion des Rohproduktes in Eiswasser mit Methylenchlorid, dreimaligem Auswaschen der organischen Phase mit Wasser, trocknen und einengen.
Durch fraktionierte Kristallisation des als Kristall-Öl-Gemisch anfallenden cis,trans-Isomerengemisches aus Äthanol, Isolieren der schwerstlöslichen Fraktion und zwei- bis dreimaliger Wiederholung dieser Operation mit den jeweils schwerstlöslichen Fraktionen konnte das reine trans-Isomere mit Schmp. 142—143°C erhalten werden (Analyse durch 270 MHz $^1$H-NMR Spektroskopie in $CDCl_3$: das bei tiefstem Feld von $\delta = 7,45$ ppm erscheinende Dublett von $H_6$ besitzt eine m-Kopplung, die beim cis-Isomeren naturgemäß nicht auftritt).

b) cis,trans-10-Brom-2-chlor-5-cyanmethylen-10,11-dihydro-dibenzo[a,d]-cyclohepten: Synthese analog Beispiel 2. Farblose Kristalle mit Schmp. 142—145°C (aus Äthanol).

c) cis,trans-2-Chlor-5-cyanmethylen-10-(4-methyl-piperazin-1-yl)-10,11-dihydro-dibenzo[a,d]-cyclohepten: Synthese analog Beispiel 1. Nach Säulenchromatographie (Kieselgel, Methylenchlorid/Methanol 95/5), farblose Kristalle mit Schmp. 77—80°C.

Beispiel 5

cis,trans-5-Cyanmethylen-10-(4-äthyl-piperazin-1-yl)-10,11-dihydro-dibenzo[a,d]-cyclohepten · ½ $H_2O$

Synthese analog Beispiel 1 unter Einsatz von N-Äthyl-piperazin. Nach Reinigung durch Säulenchromatographie (Kieselgel, Methylenchlorid/Methanol 95/5): farblose Kristalle mit Schmp. 68—71°C.

Beispiel 6

cis,trans-5-Cyanmethylen-10-(N'-methyl-homopiperazin-1-yl)-10,11-dihydro-dibenzo[a,d]-cyclohepten · ½ $H_2O$

Synthese analog Beispiel 1 unter Einsatz von N-Methyl-homopiperazin. Nach Reinigung durch Säulenchromatographie (Kieselgel, Methylenchlorid/Methanol 95/5): farblose Kristalle mit Schmp. 85—88°C.

Beispiel 7

cis,trans-5-Cyanmethylen-10-(N-$\beta$-hydroxyäthyl-piperazin-1-yl)-10,11-dihydro-dibenzo[a,d]-cyclohepten · ½ $H_2O$

Synthese analog Beispiel 1 unter Einsatz von N-$\beta$-Hydroxyäthyl-piperazin. Nach Reinigung durch Säulenchromatographie (Kieselgel, Methylenchlorid/Methanol 95/5): farblose Kristalle mit Schmp. 75—78°C.

Beispiel 8

cis, trans-5-Cyanmethylen-10-(4-methyl-4-oxy-piperazin-1-yl)-10,11-dihydro-dibenzo[a,d]-cyclohepten · ½ $H_2O$

2,4 g (7,3 mM) cis,trans-5-Cyanmethylen-10-(4-methyl-piperazin-1-yl)-10,11-dihydro-dibenzo[a,d]-cyclohepten (Beispiel 1) wird in 100 ml heißem Äthanol gelöst und mit 5 ml 30%igem Wasserstoffperoxid versetzt. Nach 5stündigem Rückflußkochen zerstört man das überschüssige Wasserstoffperoxid mit Hilfe eines kleinen Platinblechs, das in das Reaktionsgemisch geworfen wird, durch 2stündiges Rückflußkochen. Nach Filtrieren engt man das Reaktionsgemisch ein und reinigt das erhaltene N-Oxid durch Säulenchromatographie (Kieselgel, Laufmittel Methylenchlorid/Methanol 95/5). Man

isoliert 1,5 g (60%) farblose Kristalle mit Schmp. 78–80°C.
Pharmazeutische Zubereitungen, die in üblicher Weise hergestellt werden:

Beispiele für Tabletten

1.

| | |
|---|---|
| Ein Wirkstoff der Formel (I) | 5 mg |
| Lactose | 200 mg |
| Methylcellulose | 15 mg |
| Maisstärke | 50 mg |
| Talkum | 11 mg |
| Magnesiumstearat | 4 mg |

2.

| | |
|---|---|
| Ein Wirkstoff der Formel (I) | 20 mg |
| Lactose | 178 mg |
| Avicel | 80 mg |
| Polywachs 6000 | 20 mg |
| Magnesiumstearat | 2 mg |

3.

| | |
|---|---|
| Ein Wirkstoff der Formel (I) | 50 mg |
| Polyvinylpyrrolidon (mittl. M.G. 25 000) | 170 mg |
| Polyäthylenglykol (mittl. M.G. 4 000) | 14 mg |
| Hydroxypropylmethylcellulose | 40 mg |
| Talkum | 4 mg |
| Magnesiumstearat | 2 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G.: 4000), Hydroxypropylmethylcellulose, Talkum und Magnesiumstearat vermischt und zu Tabletten à 280 mg verpreßt.

4. Beispiel für Dragees

| | |
|---|---|
| Ein Wirkstoff der Formel (I) | 60 mg |
| Lactose | 90 mg |
| Maisstärke | 60 mg |
| Polyvinylpyrrolidon | 6 mg |
| Magnesiumstearat | 10 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50°C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum bestehen.

5. Kapselformulierung

| | |
|---|---|
| Ein Wirkstoff der Formel (I) | 5 mg |
| Magnesiumstearat | 2,0 mg |
| Milchzucker | 19,3 mg |

6. Injektionslösung

| | |
|---|---|
| Ein Wirkstoff der Formel (I) | 10 mg |
| Natriumchlorid | 9 mg |
| destilliertes Wasser, q.s. auf 1,0 ml | |

## Patentansprüche

1. 10-substituierte 5-Cyanmethylen-10,11-dihydro-dibenzo[a,d]-cycloheptene der allgemeinen Formel (I)

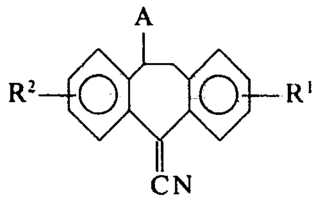

(I)

in der $R^1$ und $R^2$ Wasserstoffatome, Halogenatome, Alkylreste mit 1 bis 3 C-Atomen oder Trifluormethyl bedeuten, und für A ein Piperazinyl- oder Homopiperazinylrest steht, wobei das 2. Stickstoffatom gegebenenfalls durch einen Alkylrest mit 1 bis 3 C-Atomen, 2-Hydroxyäthyl, einen Cycloalkyl- oder Cycloalkylmethylrest mit 3 bis 7 C-Atomen im Cycloalkylring, einen Prop-2-inylrest und gegebenenfalls zusätzlich durch Sauerstoff in Form eines N-Oxids substituiert ist, und ihre physiologisch verträglichen Säureadditionssalze.

2. Verbindungen der Formel (I) nach Anspruch 1, in denen $R^1$ und $R^2$ Wasserstoff oder Chlor bedeuten und für A ein Piperazin- oder Homopiperazin-Rest, der am Ringstickstoffatom durch Methyl, Äthyl, 2-Hydroxyäthyl, Cyclopropyl oder Prop-2-inyl substituiert und gegebenenfalls in Form des N-Oxids vorliegend, steht.

3. Verbindungen der Formel (I) nach Anspruch 1, in denen $R^1$ und $R^2$ Wasserstoff oder Chlor bedeuten, und für A 4-Methyl-piperazin-1-yl, 4-(2-Hydroxyäthyl)-piperazin-1-yl, 4-Äthyl-piperazin-1-yl, 4-Methyl-4-oxy-piperazin-1-yl oder N'-Methyl-homopiperazin-1-yl steht.

4. cis,trans-5-Cyanmethylen-10-(4-methyl-piperazin-1-yl)-10,11-dihydro-dibenzo[a,d]-cyclohepten.

5. cis,trans-5-Cyanmethylen-10-(4-methyl-4-oxy-piperazin-1-yl)-10,11-dihydro-dibenzo[a,d]-cyclohepten.

6. cis,trans-5-Cyanmethylen-10-[4-(2-hydroxyäthyl)-piperazin-1-yl]-10,11-dihydro-dibenzo[a,d]-cyclohepten.

7. cis,trans-5-Cyanmethylen-10-(4-äthyl-piperazin-1-yl)-10,11-dihydro-dibenzo[a,d]-cyclohepten.

8. cis,trans-5-Cyanmethylen-2-chlor-10-(4-methyl-piperazin-1-yl)-10,11-dihydro-dibenzo[a,d]-cyclohepten.

9. cis,trans-5-Cyanmethylen-10-(N'-methyl-homopiperazin-1-yl)-10,11-dihydro-dibenzo[a,d]-cyclohepten.

10. Verfahren zur Herstellung von Verbindungen der Formel I, nach Anspruch 1, dadurch gekennzeichnet, daß man eine Verbindung der Formel (II)

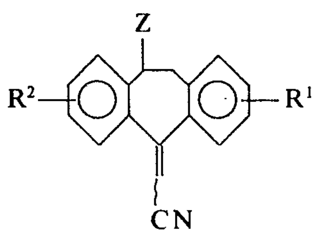

(II)

in der $R^1$ und $R^2$ die für Formel (I) gemäß Anspruch 1 angegebenen Bedeutungen haben und Z Brom oder Chlor darstellt, mit einem Nukleophil AH, in dem A die für Formel (I) angegebenen Bedeutungen hat, umsetzt und gegebenenfalls die erhaltene Verbindung in das N-Oxid und/oder in das Säureadditionssalz einer physiologisch verträglichen Säure überführt.

11. Therapeutisches Mittel, gekennzeichnet durch einen Gehalt an einer Verbindung der Formel (I) nach Anspruch 1 oder deren pharmakologisch verträgliches Säureadditionssalz als Wirkstoff neben üblichen Träger- und Verdünnungsmitteln.

12. Eine Verbindung der Formel (I) nach Anspruch 1 zur Verwendung als Sedativum, Hypnotikum, Tranquilizer oder Neurolepticum.

**Claims**

1. A 10-substituted 5-cyanomethylene-10,11-dihydrodibenzo[a,d]-cycloheptene of the general formula (I)

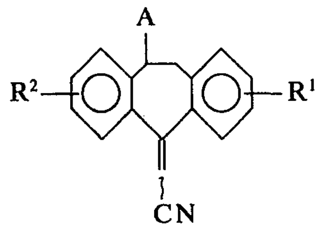

(I)

where $R^1$ and $R^2$ are hydrogen, halogen, alkyl of 1 to 3 carbon atoms or trifluoromethyl, and A is piperazinyl or homopiperazinyl, the second nitrogen atom being unsubstituted or substituted by alkyl of 1 to 3 carbon atoms, 2-hydroxyethyl, cycloalkyl or cycloalkylmethyl, each having 3 to 7 carbon atoms in the cycloalkyl ring, or prop-2-ynyl of 2 to 5 carbon atoms and being additionally, where appropriate, substituted by oxygen in the form of an N-oxide, and its physiologically tolerated addition salts with acids.

2. A compound of the formula (I) as claimed in claim 1, where $R^1$ and $R^2$ are hydrogen or chlorine and A is a piperazine or homopiperazine radical which is substituted at the ring nitrogen by methyl, ethyl, 2-hydroxyethyl, cyclopropyl or prop-2-ynyl and which may or may not be in the form of the N-oxide.

3. A compound of the formula (I) as claimed in claim 1, where $R^1$ and $R^2$ are hydrogen or chlorine and A is 4-methyl-piperazin-1-yl, 4-(2-hydroxyethyl)-piperazin-1-yl, 4-ethyl-piperazin-1-yl, 4-methyl-4-oxy-piperazin-1-yl or N'-methyl-homopiperazin-1-yl.

4. cis,trans-5-Cyanomethylene-10-(4-methyl-piperazin-1-yl)-10,11-dihydro-dibenzo[a,d]-cycloheptene.

5. cis,trans-5-Cyanomethylene-10-(4-methyl-4-oxy-piperazin-1-yl)-10,11-dihydro-dibenzo[a,d]-cycloheptene.

6. cis,trans-5-Cyanomethylene-10-[4-(2-hydroxyethyl)-piperazin-1-yl]-10,11-dihydro-dibenzo-[a,d]-cycloheptene.

7. cis,trans-5-cyanomethylene-10-(4-ethyl-piperazin-1-yl)-10,11-dihydro-dibenzo[a,d]-cycloheptene.

8. cis,trans-5-Cyanomethylene-2-chloro-10-(4-methyl-piperazin-1-yl)-10,11-dihydro-dibenzo-[a,d]-cycloheptene.

9. cis,trans-5-Cyanomethylene-10-(N'-methyl-homopiperazin-1-yl)-10,11-dihydro-dibenzo[a,d]-cycloheptene.

10. A process for the preparation of a compound of the formula (I) as claimed in claim 1, wherein a compound of the formula (II)

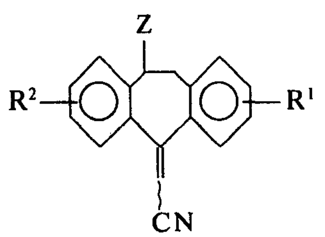

(II)

where $R^1$ and $R^2$ have the meanings given for formula (I) in claim 1 and Z is bromine or chlorine, is reacted with a nucleophilic agent AH, where A has the meanings given for formula (I), and, if desired, the compound obtained is converted into its N-oxide and/or into an addition salt with a physiologically tolerated acid.

11. A therapeutic agent, which contains a compound of the formula (I) as claimed in claim 1, or a pharmacologically tolerated acid addition salt thereof, as the acitve compound, in addition to conventional carriers and diluents.

12. A compound of the formula (I) as claimed in claim 1 for use as a sedative, hypnotic, tranquilizer or neuroleptic.

**Revendications**

1. 5-cyanométhylen-10,11-dihydro-di-benzo[a,d]-cycloheptène substitué en 10, de formule générale (I)

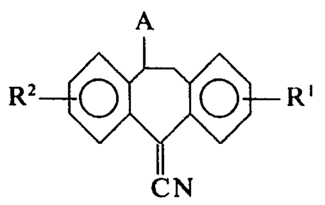

(I)

dans laquelle $R^1$ et $R^2$ représentent des atomes d'hydrogène, des atomes d'halogène, des restes alkyle ayant 1 à 3 atomes C ou du trifluorométhyle et A représente un reste pipérazinyle ou homopipérazinyle, l'atome d'azote 2 étant éventuellement substitué par un reste alkyle ayant 1 à 3 atomes C, 2-hydroxyéthyle, un reste cycloalkyle ou cycloalkylméthyle ayant 3 à 7 atomes C dans le noyau cycloalkyle, un reste prop-2-inyle et, éventuellement, en plus, par de l'oxygène sous forme d'un N-oxyde, et leurs sels d'addition d'acide acceptables physiologiquement.

2. Composés de formule (I) selon la revendication 1, dans lesquels $R^1$ et $R^2$ représentent hydrogène ou chlore, et A représente un reste pipérazine ou homopipérazine qui a été substitué, sur l'atome d'azote du noyau, par méthyle, éthyle, 2-hydroxyéthyle, cyclopropyle ou prop-2-inyle et se trouve éventuellement sous forme de N-oxyde.

3. Composés de formule (I) selon la revendication 1, dans lesquels $R^1$ et $R^2$ représentent hydrogène ou chlore, et A représente 4-méthyl-pipérazin-1-yle, 4-(2-hydroxyéthyl)-pipérazin-1-yle, 4-éthyl-pipérazin-1-yle, 4-méthyl-4-oxy-pipérazin-1-yle ou N'-méthyl-homopipérazin-1-yle.

4. cis,trans-5-cyanométhylen-10-(4-méthyl-pipérazin-1-yl)-10,11-dihydro-dibenzo[a,d]-cycloheptène.

5. cis,trans-5-cyanométhylen-10-(4-méthyl-4-oxy-pipérazin-1-yl)-10,11-dihydro-dibenzo[a,d]-cycloheptène.

6. cis,trans-5-cyanométhylen-10-[4-(2-hydroxyéthyl)-pipérazin-1-yl]-10,11-dihydro-dibenzo[a,d]-cycloheptène.

7. cis,trans-5-cyanométhylen-10-(4-éthyl-pipérazin-1-yl)-10,1-dihydro-dibenzo[a,d]-cycloheptène.

8. cis,trans-5-cyanométhylen-2-chloro-10-(4-méthyl-pipérazin-1-yl)-10,11-dihydro-dibenzo[a,d]-cycloheptène.

9. cis,trans-5-cyanométhylen-10-(N'-méthyl-homopipérazin-1-yl)-10,11-dihydro-dibenzo[a,d]-cycloheptène.

10. Procédé de préparation de composés de formule I selon la revendication 1, caractérisé par le fait que l'on fait réagir un composé de formule (II)

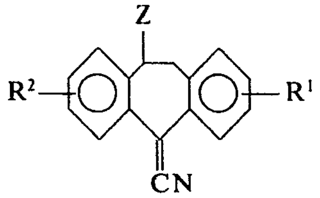

(II)

dans laquelle $R^1$ et $R^2$ ont les significations données pour la formule (I) selon la revendication 1 et Z représente brome ou chlore, avec un nucléophile AH, où A a les significations indiquées pour la formule (I) et l'on transforme éventuellement le composé obtenu en le N-oxyde et/ou en le sel d'addition d'acide d'un acide acceptable physiologiquement.

11. Agent thérapeutique caractérisé par le fait qu'il contient, comme principe actif, un composé de formule (I) selon la revendication 1 ou son sel d'addition d'acide acceptable du point de vue pharmacologie, outre des véhicules et diluants usuels.

12. Un composé de formule (I) selon la revendication 1 pour utilisation comme sédatif, hypnotique, tranquillisant ou neuroleptique.